# EUROPEAN PATENT APPLICATION

(11) **EP 4 721 665 A1**
(43) Date of publication of application: **08.04.2026**
(21) Application number: 25203908.6
(22) Date of filing: 23.09.2025
(51) Int. Cl.: A61B 5/291, A61B 5/00, G16H 50/20

(54) **METHODS AND SYSTEMS FOR CHANNEL IDENTIFICATION USING MANIFOLDS OF AN EAR-ELECTROENCEPHALOGRAPHY (EAR-EEG) SIGNAL**

(30) Priority: 04.10.2024 IN 202421075352
(71) Applicant: Tata Consultancy Services Limited, Maharashtra (IN)
(72) Inventor: MURALIDHARAN, Kartik, 560066 Bangalore, Karnataka (IN); JAYAS, Tanuja, 560066 Bangalore, Karnataka (IN); GUBBI LAKSHMINARASIMHA, Jayavardhana Rama, 560009 Bangalore, Karnataka (IN); ANAND, Adarsh, 560009 Bangalore, Karnataka (IN); RAJALINGAM, Jhanavi, 560066 Bangalore, Karnataka (IN); KUMAR, Bhartendu, 400001 Mumbai, Maharashtra (IN); BANGALORE SAMPATHKUMAR, Vivek, 560066 Bangalore, Karnataka (IN); PAL, Arpan, 700091 Kolkata, West Bengal (IN)
(74) Representative: Goddar, Heinz J.

(57) **Abstract**

Brain monitoring is performed using an Ear-electroencephalography (Ear-EEG) which records synchronized electrical behaviour of neurons in brain at high temporal resolution using a wearable ear device. For proper functioning of the wearable ear device, sensor electrodes need to be within the ear to remain in constant contact with the skin. However, body movements tend to disrupt extent of contact, leading to noisy signals being captured, which are often difficult to distinguish from a valid EEG signal. Thus, identification of channels that are capturing EEG is necessary. The present disclosure provides a method and system for channel identification using manifolds of an Ear-EEG signal. In the present disclosure, a plurality of manifold features are extracted from a signal received from the wearable ear device. The plurality of manifold features are further classified as EEG and non-EEG channels using an unsupervised clustering model across real-world stimuli in the wearable ear device.

## Description

### CROSS-REFERENCE TO RELATED APPLICATIONS AND PRIORITY

The present application claims priority to Indian application no. 202421075352 filed on October 4, 2024.

### TECHNICAL FIELD

The disclosure herein generally relates to the field of Ear-EEG signal processing, and, more particularly, to methods and systems for channel identification using manifolds of an Ear-electroencephalography (Ear-EEG) signal.

### BACKGROUND

The brain is a next frontier in everyday human sensing. Multiple non-invasive imaging tools such as electroencephalography (EEG), magnetoencephalography (MEG), near-infrared spectroscopy (NIRS), and functional magnetic resonance imaging (fMRI) are being utilized to study the brain function and develop related applications. Among these, EEG is a method of recording the synchronized electrical behaviour of neurons in the brain at high temporal resolution. EEG is a well-established tool in neurophysiology for diagnosis of various neurological disorders as well as for creating brain computer interfacing (BCI) paradigms. Currently, recording of EEG requires use of devices to be worn on scalp, with data collection limited in a stationary and controlled environment. In contrast, Ear-EEG is an approach of capturing EEG using electrodes placed inside the ear. In order for an ear able to function, it is necessary for sensor electrodes within the ear to remain in constant contact with the skin. However, body movements tend to disrupt the extent of contact, leading to noisy signals being captured, which are often difficult to distinguish from a valid EEG signal.

There exist few works analyzing feasibility of wearable EEG and ensuring an accurate analysis to select channels that contain EEG signals. Typically, selection of EEG channels is performed based on visual inspection. However, this is a time-consuming and subjective procedure. In a conventional approach, electrode-to-skin contact was determined using an impedance value at a beginning of an experiment for an electrode, and if the impedance values were lower, only those channels would be selected. Although this conventional approach verifies an initial electrode-skin contact, it is unsuitable for persistently checking electrode connectivity. Signal amplitude is also used as a feature for identifying and selecting channels that had amplitude lower than 500 *µ*Volts. However, these approaches are suitable for an in-lab, post analysis, and scalp EEG channel selection

Other conventional approaches for EEG channel selection include observing a response generated by a stimulus like in an Auditory Steady State Response (ASSR) paradigm. With ASSR, channels where a peak at 40 Hz was seen, are selected for further analysis. While this method works, the stimulus used can still be unpleasant to hear. Another method based on alpha power and variance was used for closed eyes to ensure connection of electrode before proceeding with EEG data collection. Further, a few channel rejection techniques are used for the validation of EEG. However, these techniques are not generalized for different environments and overlook dynamic nature of the EEG signals. Further, few conventional features such as kurtosis, periodogram, Auto-correlation function (ACF), and auto-regression based modelling has been used to identify atypical behaviour in EEG signals. However, these conventional features-based modelling does not show any significant difference between EEG and non-EEG signals.

### SUMMARY

Embodiments of the present disclosure present technological improvements as solutions to one or more of the above-mentioned technical problems recognized by the inventors in conventional systems. For example, in one embodiment, a processor implemented method is provided. The processor implemented method, comprising: receiving in real time, via one or more hardware processors, a first signal and a second signal generated as a neural response of a user to at least one of a plurality of stimuli actions from each of a plurality of channels within a wearable Ear-electroencephalography (Ear-EEG) device worn by the user, wherein the plurality of stimuli actions are indicative of a context from day-to-day activities of the user obtained automatically from the wearable Ear-EEG device, and wherein the second signal is indicative of a resting or a non-resting state of the user; preprocessing, via the one or more hardware processors, one of: (i) the first signal and (ii) the second signal using a plurality of preprocessing techniques to obtain a preprocessed signal; segmenting, via the one or more hardware processors, the preprocessed signal into a plurality of data segments following an onset of the second signal, wherein the plurality of data segments indicate epochs of a plurality of window lengths; extracting, via the one or more hardware processors, a plurality of manifold features from each of the plurality of data segments, wherein the plurality of manifold features support in (i) determining a structure of data and (ii) identifying one or more complex dynamic characteristics of the first signal the second signal; classifying, via the one or more hardware processors, each of the plurality of data segments into (i) a first category and a (ii) a second category based on the plurality of manifold features using an unsupervised clustering model, wherein the first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data; and selecting, via the one or more hardware processors, the plurality of data segments classified into the first category to identify the first set of channels which capture the electroencephalogram (EEG) data for monitoring a status representing health condition of the user.

In another aspect, a system is provided. The system comprising a memory storing instructions; one or more communication interfaces; and one or more hardware processors coupled to the memory via the one or more communication interfaces, wherein the one or more hardware processors are configured by the instructions to: receive in real time, a first signal and a second signal generated as a neural response of a user to at least one of a plurality of stimuli actions from each of a plurality of channels within a wearable Ear-electroencephalography (Ear-EEG) device worn by the user, wherein the plurality of stimuli actions are indicative of a context from day-to-day activities of the user obtained automatically from the wearable Ear-EEG device, and wherein the second signal is indicative of a resting or a non-resting state of the user; preprocessing one of: (i) the first signal and (ii) the second signal using a plurality of preprocessing techniques to obtain a preprocessed signal; segment the preprocessed signal into a plurality of data segments following an onset of the second signal, wherein the plurality of data segments indicate epochs of a plurality of window lengths; extract a plurality of manifold features from each of the plurality of data segments, wherein the plurality of manifold features support in (i) determining a structure of data and (ii) identifying one or more complex dynamic characteristics of the first signal the second signal; classify each of the plurality of data segments into (i) a first category and a (ii) a second category based on the plurality of manifold features using an unsupervised clustering model, wherein the first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data; and select the plurality of data segments classified into the first category to identify the first set of channels which capture the electroencephalogram (EEG) data for monitoring a status representing health condition of the user.

In yet another aspect, a non-transitory computer readable medium is provided. The non-transitory computer readable medium are configured by instructions for receiving in real time, a first signal and a second signal generated as a neural response of a user to at least one of a plurality of stimuli actions from each of a plurality of channels within a wearable Ear-electroencephalography (Ear-EEG) device worn by the user, wherein the plurality of stimuli actions are indicative of a context from day-to-day activities of the user obtained automatically from the wearable Ear-EEG device, and wherein the second signal is indicative of a resting or a non-resting state of the user; preprocessing one of: (i) the first signal and (ii) the second signal using a plurality of preprocessing techniques to obtain a preprocessed signal; segmenting the preprocessed signal into a plurality of data segments following an onset of the second signal, wherein the plurality of data segments indicate epochs of a plurality of window lengths; extracting a plurality of manifold features from each of the plurality of data segments, wherein the plurality of manifold features support in (i) determining a structure of data and (ii) identifying one or more complex dynamic characteristics of the first signal the second signal; classifying each of the plurality of data segments into (i) a first category and a (ii) a second category based on the plurality of manifold features using an unsupervised clustering model, wherein the first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data; and selecting the plurality of data segments classified into the first category to identify the first set of channels which capture the electroencephalogram (EEG) data for monitoring a status representing health condition of the user.

In accordance with an embodiment of the present disclosure, the first signal is a continuous signal obtained from the wearable Ear-EEG device.

In accordance with an embodiment of the present disclosure, the second signal is a modified form of the first signal obtained from the wearable Ear-EEG device when the context from the day-to-day activities of the user is obtained automatically from the wearable Ear-EEG device.

In accordance with an embodiment of the present disclosure, the second signal is an event based intermittent signal.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for channel identification using manifolds of an Ear-electroencephalography (Ear-EEG) signal, according to some embodiments of the present disclosure.
FIG. 2, with reference to FIG. 1, illustrates an exemplary flow diagram illustrating a method for channel identification using manifolds of an Ear-electroencephalography (Ear-EEG) signal, using the system of FIG. 1, in accordance with some embodiments of the present disclosure.
FIGS. 3A and 3B illustrate a pictorial representation of placement of an example customized earpiece with electrodes for channel identification using manifolds of an Ear-electroencephalography (Ear-EEG) signal, according to some embodiments of the present disclosure.
FIG. 4 illustrates an example protocol with a plurality of stimuli presented in a random sequence on different days for channel identification using manifolds of an Ear-electroencephalography (Ear-EEG) signal, according to some embodiments of the present disclosure.
FIG. 5 depicts a graphical representation illustrating an observed attenuation in power in alpha band for channel identification using manifolds of an Ear-electroencephalography (Ear-EEG) signal, according to some embodiments of the present disclosure.
FIG. 6 depicts a graphical representation illustrating number of channels validated as EEG and non-EEG using EOEC paradigm, according to some embodiments of the present disclosure.
FIGS. 7A through 7C depict graphical representations illustrating mean value of the plurality of manifold features obtained from manifold analysis for EEG and non-EEG channels for different window lengths, according to some embodiments of the present disclosure.
FIGS. 8A and 8B depict graphical representations illustrating performance metrics for classification of channels of the wearable device into EEG and non-EEG using manifold analysis and BGM clustering performed on a subject for two different window lengths, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

Brain is the most important part of a human body, and hence study of brain functioning is essential. For studying the brain function and developing related applications, multiple non-invasive imaging tools such as EEG, magnetoencephalography (MEG), near-infrared spectroscopy (NIRS), and functional magnetic resonance imaging (fMRI) are being used. Among these, EEG is a method of recording the synchronized electrical behaviour of the neurons in the brain at high temporal resolution. EEG is a well-established tool in neurophysiology for the diagnosis of various neurological disorders as well as for creating brain computer interfacing (BCI) paradigms.

Currently, recording of EEG requires use of devices to be worn on the scalp, with data collection limited in a stationary and controlled environment. In contrast, Ear-EEG is an approach of capturing EEG using electrodes placed inside the ear. Collecting data using electrodes in the ear is a more comfortable and user-friendly approach, as compared to the scalp, and can be easily integrated into our everyday activities. Ear-EEG also allows recording in-situ data for longer spans of time, and thus finds promising use in a wide range of longitudinal applications such as stress, attention tracking, sleep monitoring, and/or the like. But collecting EEG from within the ear does have its own limitations. Fundamentally, EEG signals reflect an oscillatory activity of the brain, which are broadly classified into the five canonical frequency bands. These bands are delta (1-4 Hz), theta (4-8 Hz), alpha (8-12 Hz), beta (12- 30 Hz), and gamma (30 - 45 Hz), with the amplitude ranging between 20 to 100 micro-Volts. While Ear-EEG has slightly less amplitude range, the signal-to-noise ratio (SNR) is still similar to that of scalp EEG. It is observed that there is a necessity to always receive signals with above characteristics when capturing EEG from within the ear. Further, constant body movements can alter a number of electrodes picking up EEG and extent of the electrode surface that is in contact with the skin, aggravating in-ear EEG data collection. Signals collected under these conditions are noisy and often difficult to distinguish from real EEG signals.

Thus, there is a need for a technique that interprets characteristics of the Ear-EEG signal and distinguishes EEG signals from non-EEG signals. EEG signals possess inherent non-linear and chaotic behaviour due to physiological phenomenon and neuronal activity in the brain. They are quasi-stationary, which means they are stationary only in short time intervals and exhibit change in signal distribution which can be measured in terms of both Gaussian process and deviation from Gaussian distribution.

The present disclosure addresses the unresolved problems of the conventional approaches by exploring role of manifolds in assessing structure of the EEG signal. Manifold analysis helps in understanding the structure of the data and identifying complex dynamics of the EEG signal. The intrinsic dimension of a manifold is a key parameter, where if the dimension is small, it means that important information of the data lies on the same dimension, but if the dimension is too high, that means the projection can be noisy and unstable. Furthermore, curvature assessment of a manifold indicates the dynamic changes that occur in the bends and twists of a geometry.

Embodiments of the present disclosure provides a method and system for channel identification using manifolds of an Ear-electroencephalography (Ear-EEG) signal. In the present disclosure, an in-situ method for the selection of channels that capture EEG signals from ear wearables is provided. Furthermore, an ability of these manifolds I tested using a clustering algorithm to classify EEG and non-EEG channels and achieve an accuracy of 87.09% for the classification. In other words, the present disclosure performs channel selection using geometrical features evaluated on the manifolds of EEG data. The performance of the classification of channels into 'EEG' and 'non-EEG' signals using the dimension and curvature of manifold is also provided. The method of the present disclosure helps in enhancing the performance of various applications pertaining to EEG monitoring and processing.

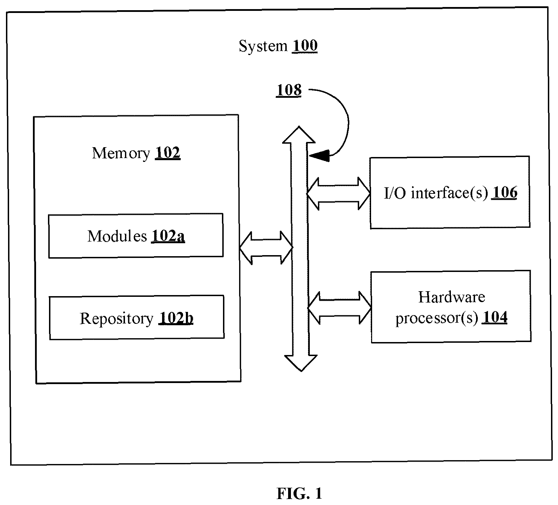

Referring now to the drawings, and more particularly to FIGS. 1 through 10, where similar reference characters denote corresponding features consistently throughout the figures, there are shown preferred embodiments, and these embodiments are described in the context of the following exemplary system and/or method.

FIG. 1 illustrates an exemplary system for channel identification using manifolds of an Ear-electroencephalography (Ear-EEG) signal, according to some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface, and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computer, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, new data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

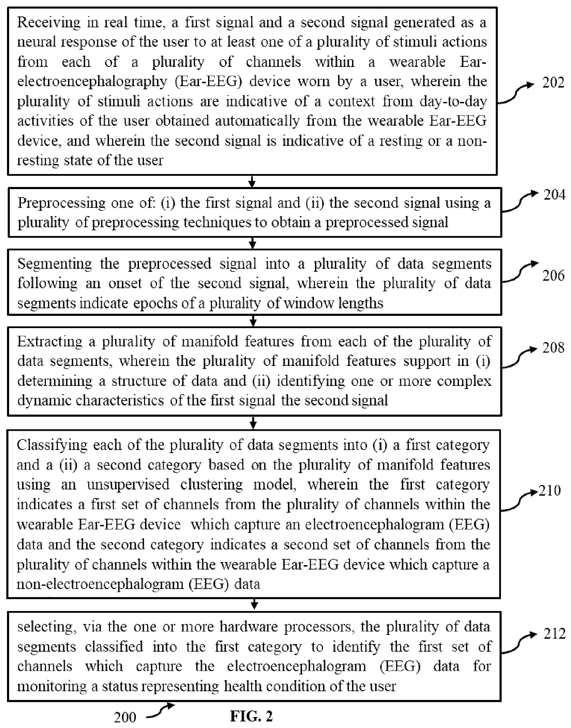

FIG. 2, with reference to FIG. 1, illustrates an exemplary flow diagram illustrating a method for channel identification using manifolds of an Ear-EEG signal, using the system 100 of FIG. 1, in accordance with some embodiments of the present disclosure.

Referring to FIG. 2, in an embodiment, the system(s) 100 comprises one or more data storage devices or the memory 102 operatively coupled to the one or more hardware processors 104 and is configured to store instructions for execution of steps of the method by the one or more processors 104. The steps of the method 200 of the present disclosure will now be explained with reference to components of the system 100 of FIG. 1, the flow diagram as depicted in FIG. 2, and one or more examples. Although steps of the method 200 including process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

In an embodiment, at step 202 of the present disclosure, one or more hardware processors 104 are configured to receive in real time, a first signal and a second signal generated as a neural response of a user to at least one of a plurality of stimuli actions from each of a plurality of channels within a wearable Ear-electroencephalography (Ear-EEG) device worn by the user. The plurality of stimuli actions are indicative of a context from day-to-day activities of the user obtained automatically from the wearable Ear-EEG device. The wearable Ear-EEG device could be a customized earpiece with a specific number of electrodes (i.e., interchangeably referred as channels throughout the description) placed in the ear of the user. The user could a subject such as a patient whose brain activity needs to be monitored. The first signal refers to raw signal obtained from the subject wearing the wearable Ear-EEG device. The first signal a continuous signal obtained from the wearable Ear-EEG device. In embodiment, the second signal is an event based intermittent signal. The second signal is a modified form of the first signal obtained from the wearable Ear-EEG device when the context from the day-to-day activities of the user is obtained automatically from the wearable Ear-EEG device. In an embodiment, the second signal is indicative of a resting (e.g., a first state) or a non-resting state (e.g., a second state) of the user.

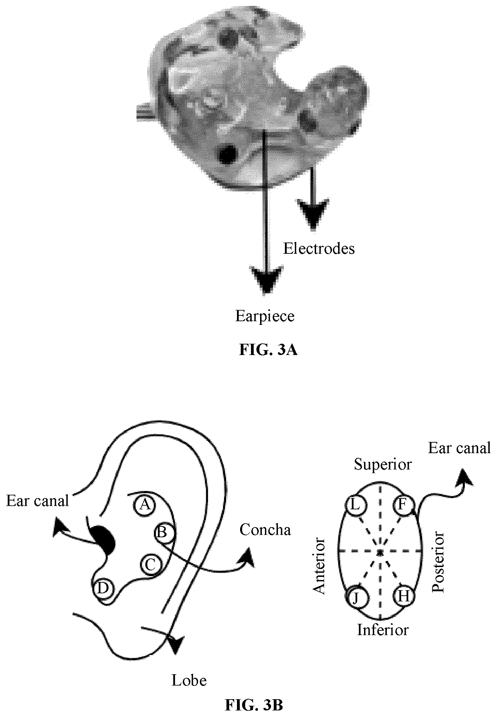

In the context of present disclosure, an experiment was performed for signal acquisition where four subjects including 3 males and 1 female aged 30.25 ± 6.8 participated after informed consent for four days. The customized earpieces with eight electrodes were placed in each ear of each of the four subjects, where four electrodes were used in concha represented as xA, xB, xC, xD and four in canal represented as xF, xH, xJ, xL, for capturing the first signal from the ear. FIGS. 3A and 3B illustrate a pictorial representation of placement of an example customized earpiece with electrodes for channel identification using manifolds of an Ear-EEG signal, according to some embodiments of the present disclosure. FIG. 3A provides a pictorial representation of an example customized earpiece with electrodes for channel identification using manifolds of an Ear-EEG signal, according to some embodiments of the present disclosure. FIG. 3B illustrates a cross-sectional view of a left ear depicting regions of electrode contact in the customized earpiece of FIG. 3A, according to some embodiments of the present disclosure. As shown in FIG. 3B, labels A, B, C, and D correspond/pertain to concha (external ear), and labels F, J, H, and L correspond//pertain to the canal (inner ear) electrodes.

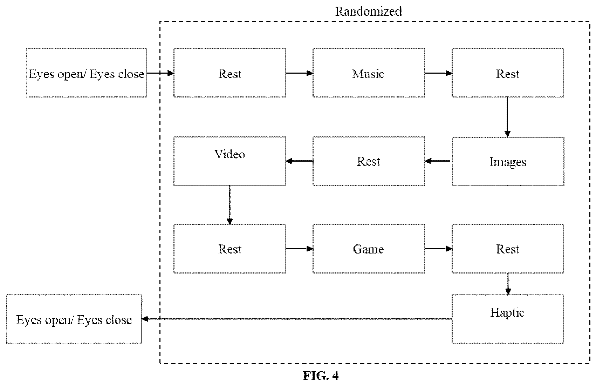

In the experiment performed, two OpenBCI Cyton^{®} boards were used to capture the first signal from each of the plurality of channels at a sampling rate of 250 Hertz (Hz). It is to be understood by a person having ordinary skill in the art or person skilled in the art that such sampling rate shall not be construed as limiting the scope of the present disclosure. An ipsilateral reference configuration was used for the recording, with the reference and bias electrodes placed on respective earlobes. A mobile phone was used to administer a plurality of stimuli, as opposed to a desktop, as the mobile phone presents a more natural environment and is more likely to be used. This made it important to observe whether a stimulus presented on a phone would elicit a neural response. In the experiment performed, a protocol comprising five different types of stimuli, including audio, video, image, game, and haptic response, was taken into consideration from real-life day to day activities to validate presence of EEG in the plurality of channels. FIG. 4 illustrates an example protocol with a plurality of stimuli presented in a random sequence on different days for channel identification using manifolds of an Ear-EEG signal, according to some embodiments of the present disclosure. As shown in FIG. 4, an entire recording was done in 11 minutes and 10 seconds for one subject. Table 2 below provides details of the plurality of stimuli presented with their time duration.

**Table 1**

| Stimuli | Duration | Real-world examples |
|---|---|---|
| Audio | 90 sec | Ringtone, alarm, horn, doorbell, songs, phone calls, podcasts, radio |
| Video | 60 sec | TV, movies, Social media videos |
| Images | 30 sec | Ads and commercials, photos |
| Haptic | 70 sec | Phone vibration, Smart watch |
| Game | 70 sec | Puzzles, Action, Strategy and Decision-making games |

In an embodiment, each type of stimulus from the plurality of stimuli in Table 1 was selected to elicit distinct neural responses so that a comprehensive assessment of the system of present disclosure in validating the presence of EEG could be done. A rest of 30 seconds was given between each stimulus. For validation of selected channels, data was collected for eyes open, eyes closed (EOEC) paradigm. The subject performed two trials, with 30 seconds of eyes open and 30 seconds of eyes closed, at the start and end of the data collection. From Table 1, following are observed:
1. Audio: The subject was presented with a 90-seconds long song or audio-book session on the mobile phone, and the subject's EEG response was recorded. Such audio stimuli cause a repeated and spontaneous excitation in an EEG waveform.
2. Video: The subject was presented with a 60-second video excerpt from movies of different genres.
3. Image: The subject was asked to swipe through some random static images for 30 seconds.
4. Haptic: A haptic response in the form of tactile feedback in EEG was recorded when the subject received a call on the phone, and a vibratory notification was sent on a smartwatch worn by the user on the wrist and connected to the phone.
5. Game: The subject was given a simple reaction time game involving visual cues to play on the phone for 60 seconds, and the somatosensory responses during this time were recorded.

At step 204 of the present disclosure, the one or more hardware processors 104 are configured to preprocess one of: (i) the first signal and (i) the second signal using a plurality of preprocessing techniques to obtain a preprocessed signal. The plurality of preprocessing techniques may include but are not limited to a filtering mechanism, and a flat channel rejection technique. In an embodiment, pre-processing was performed using a band-pass filter with a lower cut-off frequency of 0.5 Hz and a higher cut-off frequency of 45 Hz to suppress Direct Current (DC) and drifts in received input signal which could be any of the first signal or the second signal. This was followed by a notch filter at 50 Hz to suppress the powerline noise. Subsequently, channels with saturated signals because of poor connection were removed from further analysis.

Further, at step 206 of the present disclosure, the one or more hardware processors 104 are configured to segment the preprocessed signal into a plurality of data segments following an onset of the second signal. The plurality of data segments indicates epochs of different window lengths. This means that the preprocessed signal is segmented into epochs of a plurality of window lengths when the context from the day-to-day activities of the user is obtained.

Furthermore, at step 208 of the present disclosure, the one or more hardware processors 104 are configured to extract a plurality of manifold features from each of the plurality of data segments. The plurality of manifold features support in (i) determining a structure of data and (ii) identifying one or more complex dynamic characteristics of the first signal the second signal. In an embodiment, the plurality of manifold features may include but are not limited to a dimension, a volume, and a curvature characteristic of a manifold of each of the plurality of data segments of the preprocessed signal. In an embodiment, the plurality of manifold features provides an understanding of non-linearity and geometry of EEG signals over a period of time. To calculate base feature values, manifolds were implemented on each of the plurality of data segments separately for a data segment of ±N seconds at onset of a stimuli from the plurality of stimuli.

Referring to FIG. 2, at step 210 of the present disclosure, the one or more hardware processors 104 are configured to classify each of the plurality of data segments into (i) a first category and a (ii) a second category based on the plurality of manifold features using an unsupervised clustering model. The first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data.

In an embodiment, for the stimuli from the plurality of stimuli where a significant difference in the plurality of manifold features was observed, four clustering methods were used to assess an ability of the plurality of manifold features to identify EEG channels. In an embodiment, clustering methods performed by the unsupervised clustering model may include but are not limited to K-means clustering, spectral clustering, Gaussian Mixture Model (GMM), Bayesian Gaussian Mixture (BGM), and /or the like. The K-means Clustering refers to an unsupervised algorithm where data is partitioned based on a weighted sum of distance between points. The spectral clustering refers to a technique to divide data using their spatial proximity into groups that are internally similar. The Gaussian Mixture Model is a probabilistic model where clusters are characterized by a Gaussian multivariate distribution and the model weighted sum of the described distributions. The Bayesian Gaussian Mixture refers to a variation of the Gaussian mixture model where probability of a data point belonging to a cluster is defined using Bayes' theorem.

Further, at step 212 of the present disclosure, the one or more hardware processors 104 are configured to select the plurality of data segments classified into the first category to identify the first set of channels which capture the electroencephalogram (EEG) data for monitoring a status representing health condition of the user. For validation of the one or more channels identified using the method of the present disclosure, alpha attenuation was evaluated for the EOEC paradigm. The change in alpha power was computed as a ratio of absolute power in the alpha band during EO and EC. The one or more channels that show a significant increase in alpha power when the subject's eyes are closed are the channels where the Ear-EEG is actually getting recorded. These channels are labeled as 'EEG' channels and the rest as 'non-EEG'.

The steps 208 through 212 are further better understood by way of following description provided as an exemplary explanation.

In an embodiment, prior to attempting alternative techniques, first it is validated if standard signal processing based conventional features would be sufficient to differentiate between EEG and non-EEG signals. In the present disclosure, power in different frequency ranges, kurtosis, ACF, and periodogram of epochs of rest and stimuli period were measured separately. The differences between the conventional features for rest and the stimuli period were then calculated and used to assess their distinguishing ability between EEG and non-EEG signals. In order to observe if there was any difference between structure of EEG and non-EEG channels, the dimension, volume, and curvature of the manifold of these signals were evaluated. To check significant difference between dimension, curvature, and conventional features of EEG and non-EEG channels, a Mann-Whitney U test was performed for each stimulus.

In the present disclosure, the manifolds of the Ear-EEG signal were analyzed that allows to leverage complex dynamics of neural responses of everyday activities to identify channels picking up EEG data. The method of the present disclosure greatly reduces and even eliminates the time needed in visually inspecting and rejecting channels. The following observations show significance of the method of the present disclosure:
**A. Enhanced Channel Selection:** The dimension of the manifold helps in understanding degree of freedom of neural data of the subject. It is observed that EEG data had higher dimensions than non-EEG data because of more complex structure, which shows more neural information is stored on various levels of the manifolds. On the other hand, synchronized neuronal firing and repetitive signal structure causes the EEG data to have a smaller curvature than that of the non-EEG data. A reduction in accuracy was observed with longer window length suggesting that rapid EEG responses are more accurately captured in the manifolds with smaller window length. In the present disclosure, effectiveness of the plurality of manifold features in identifying the EEG data using their geometry is investigated. Different unsupervised clustering methods were implemented using the plurality of manifold features, and a robust automated method of channel selection was formulated. Furthermore, the clustering techniques used for classification are able to successfully segregate EEG and non-EEG channels based on characteristics of their manifolds. The method of the present disclosure helps in decreasing computational load on further applications where only channels with EEG data is made available for use. Also, this might help in increasing the performance of downstream tasks as only usable data will be passed on for implementation.
**B. Adaptation to Different Stimuli:** The dimension and curvature also capture information regarding the complexity of the EEG signal based on a type of stimuli used. Haptic stimulus has the lowest dimension value because of simple somatosensory processing, while audio stimulus has the highest dimension value because of complex processing of auditory signals. Furthermore, use of a combination of visual, auditory, and motor components in video and gaming stimuli leads them to have significant curvature changes to capture multiple neural activities happening. Additionally, the image stimulus has a very small curvature change, depicting a requirement of only visual cognitive functions. For video stimuli, the plurality of manifold features holds a significant difference between 'EEG' and 'non-EEG' channels for larger window length (±15 *sec*) pertaining to complex processing of visio-motor and emotional responses.
**C. Adaptation to region of the electrode placement:** The dimension and curvature of the manifolds in different regions also provide insights into spatial distribution of EEG in different regions within the ear. It is observed that the curvature of the concha region is smaller, and the dimension is higher than that of the canal region. Thus, channels in the concha region are more likely to represent true EEG behaviour, leading to a better differentiation between EEG and non-EEG channels in the concha region.
**D. Real-World Adaptability:** At its core, the method of the present disclosure uses dimensions and curvature, which involve complex calculations, and to be able to have a practical application and use them with a wearable device, it is important to know the complexity of computing. The complexity of computing the dimension of a channel in the method of the present disclosure depends on a number of channels (n), length of the data segment (*D*)*,* and number of nearest neighbours (*k*) considered. Whereas, for curvature, the complexity also depends on dimension (*d*) of the channel along with the above factors. But practically, there are machine-dependent variables, which are influenced by software and hardware factors of a machine, that affect the time taken for the evaluation of the plurality of manifold features. So, the complexity for dimension is *O*(*λ* * *n* * *D* + *ψ* * *n* * *log*(*k*)) and for curvature is *O*(*λ * n * D + ψ* * *n* * *k* * *log*(*k*) *+ µ * D² * k* + *ω * k * d⁴*), where *λ*, *ψ*, *µ,* and *ω* are machine-dependent variables. Table 2 provides the parameters for the system of the present disclosure and the time taken to calculate the plurality of manifold features.

**Table 2**

| | *λ* | *ψ* | *µ* | *ω* | Time taken |
|---|---|---|---|---|---|
| Dimension | 35.9E-6 | 17.5E-2 | - | - | 0.25 ± 0.02 s |
| Curvature | 27.6E-4 | 71.8E-6 | -6.4E-7 | -6.4E-9 | 7.45 ± 0.01 s |

### EXPERIMENTAL RESULTS

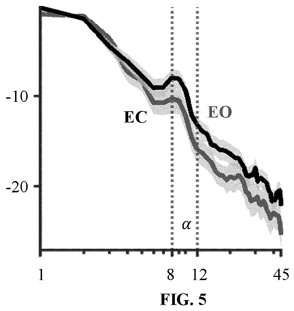

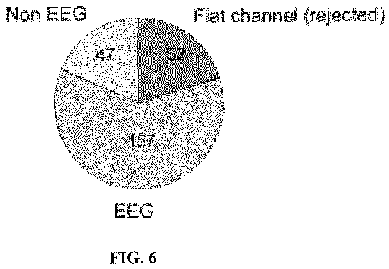

In an embodiment, the plurality of manifold features were pooled across subjects, and the split for training and testing data was 70-30%. The training features were normalized using z-score, and the same normalization parameters were used for testing the features. In an embodiment, for data validation, flat channel detection resulted in 52 channels being rejected. The labelling of each of the plurality of channels was done using the alpha attenuation seen in the eyes open eyes closed (EOEC) paradigm. FIG. 5 depicts a graphical representation illustrating an observed attenuation in power in alpha band for channel identification using manifolds of an Ear-EEG signal, according to some embodiments of the present disclosure. As shown in FIG. 5, power in EO and EC condition in an electrode is observed. FIG. 6 depicts a graphical representation illustrating number of channels validated as EEG and non-EEG using EOEC paradigm, according to some embodiments of the present disclosure. As shown in FIG. 6, a total of 157 electrodes were found to show alpha change at 10 Hz frequency from a grand total of 256 electrodes across all subjects for all days. These electrodes were considered as channels collecting EEG data or EEG signals.

In an embodiment, an analysis of conventional features and manifold feature is performed. Results of significance testing on conventional and manifold features are shown in Table 3.

**Table 3**

| **p-values** | Audio | Images | Video | Haptic | Game |
|---|---|---|---|---|---|
| Window Length | ±2 sec | ±2 sec | ±15 sec | ±2 sec | ±2 sec |
| Kurtosis | 0.79 | **0.01** | 0.99 | 0.73 | 0.61 |
| ACF | 0.70 | 0.52 | 0.32 | 0.49 | **0.02** |
| Periodogram | 0.06 | 0.4 | 0.95 | 0.25 | 0.05 |
| Dimension | **0.016** | **0.001** | **0.023** | **0.003** | **0.041** |
| Curvature | **0.01** | **0.005** | **0.01** | **0.03** | **0.001** |

As observed from Table 3, no statistical difference between EEG and non-EEG signals using the conventional features across all stimuli is observed. However, when the dimensions and the curvature of all data segments of EEG and non-EEG channels are evaluated, a significant difference (*p* < 0.05) was found between two subsets for a window length greater than ±2 seconds before and after a stimulus was presented. This was consistent across all the stimuli, except video stimuli, where a significant difference between EEG and non-EEG channels was seen at larger window lengths. Hence, a window length of ±2 seconds was chosen for calculating value of the plurality of manifold features and then using them for the classification of the channel as EEG or non-EEG channel.

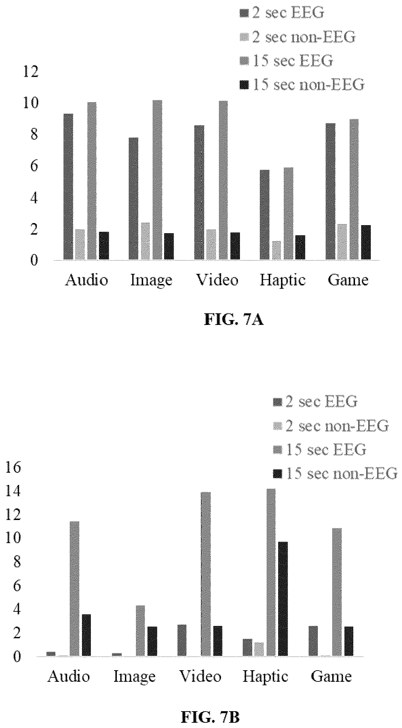

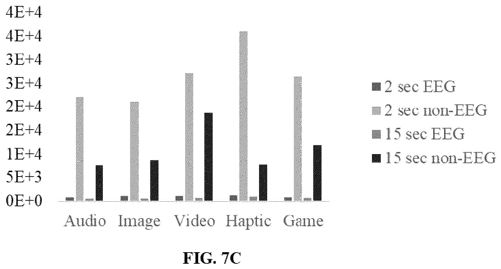

FIGS. 7A through 7C depict graphical representations illustrating mean value of the plurality of manifold features obtained from manifold analysis for EEG and non-EEG channels for different window lengths, according to some embodiments of the present disclosure. FIG. 7A depicts graphical representation illustrating mean value of dimension obtained from manifold analysis for EEG and non-EEG channels for different window lengths, according to some embodiments of the present disclosure. FIG. 7B depicts graphical representation illustrating mean value of volume obtained from manifold analysis for EEG and non-EEG channels for different window lengths, according to some embodiments of the present disclosure. FIG. 7C depicts graphical representation illustrating mean value of curvature obtained from manifold analysis for EEG and non-EEG channels for different window lengths, according to some embodiments of the present disclosure. The different window lengths chosen are ±2 and ±15 seconds respectively. As shown in FIGS. 7A through 7C, it was observed that during the manifold analysis, the dimension of EEG signals was quite higher than the non-EEG signals, while the curvature of the EEG signals was lower than the non-EEG signals. It was also observed that the volume of the channels with EEG was more than the channels without EEG data. Additionally, for volume evaluation of a manifold, all the points of the manifold are required to be used as it is a higher-level feature of the manifolds. So, volume evaluation cannot be done at a segment level, and hence, volume was not used as a feature in clustering algorithms.

Table 4 provides a classification accuracy obtained by different unsupervised clustering algorithms for different stimuli (except videos) when the window length was taken as ±2 seconds.

**Table 4**

| **Clustering Method** | Audio | Images | Haptic | Game |
|---|---|---|---|---|
| K-means | 0.81 | 0.77 | 0.81 | 0.81 |
| Spectral Clustering | 0.79 | 0.87 | 0.79 | 0.79 |
| Gaussian Mixture Model | 0.58 | 0.77 | 0.81 | 0.81 |
| Bayesian Gaussian Mixture | 0.87 | 0.79 | 0.82 | 0.79 |

As shown in Table 3, among the clustering algorithms evaluated, it was observed that the Bayesian Gaussian Mixture (BGM) clustering method demonstrated highest accuracy across all the stimuli. Here, the clustering algorithms use the dimension and the curvature of the manifold evaluated for ± 2 seconds window length as feature vectors to effectively separate EEG and non-EEG channels.

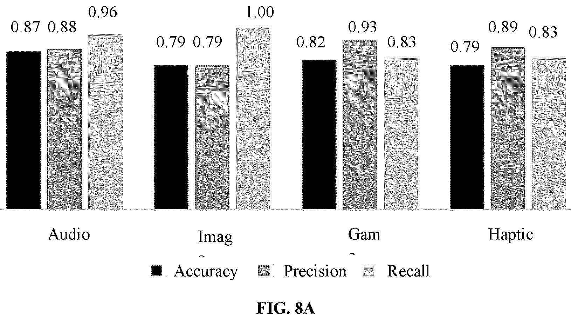

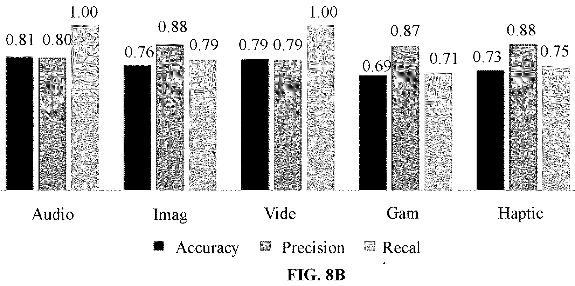

FIGS. 8A and 8B depict graphical representations illustrating performance metrics for classification of channels of the wearable device into EEG and non-EEG using manifold analysis and BGM clustering performed on a subject for two different window lengths, according to some embodiments of the present disclosure. FIG. 8A depicts graphical representations illustrating accuracy, precision, and recall respectively for classification of channels of the wearable device into EEG and non-EEG using manifold analysis and BGM clustering performed on a subject for ±2 seconds window length, according to some embodiments of the present disclosure. As observed from FIG. 8A, the BGM clustering was able to attain a highest accuracy of 87.09% for audio stimuli. When testing the performance of BGM clustering for a larger window length, it was observed that the model's accuracy decreased. FIG. 8B depicts graphical representations illustrating accuracy, precision, and recall respectively for classification of channels of the wearable device into EEG and non-EEG using manifold analysis and BGM clustering performed on a subject for ±15 seconds window length, according to some embodiments of the present disclosure. As observed from FIG. 8B, the BGM clustering was able to achieve an accuracy of 79.03% for video stimuli.

In an embodiment, to further analyze the association of the manifolds with different electrodes, the dimension and curvature of the manifolds are assessed for canal vs concha electrodes and right vs left ear electrodes. It was observed that the channels in the concha region had significantly higher dimensions and low curvature than the channels in the ear canal. For the electrodes in each ear, a significant difference was seen in dimension for audio, video, and images, while audio, images, and haptic showed a significant difference in the curvature of the manifold.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the embodiments or if they include equivalent elements with insubstantial differences from the literal language of the embodiments described herein.

The present disclosure establishes a need to identify EEG channels in a wearable ear device. Embodiment of the present disclosure provides a practical way of identifying channels from ear wearables with the help of day-to-day activities and using a robust algorithm. In the present disclosure, manifold features to differentiate between EEG and non-EEG channels across real-world stimuli in a wearable ear device are used. It is observed in the present disclosure that for capturing relevant neural responses, a window length of ±2 seconds around the stimuli is optimal, an accuracy of more than 80% is achieved for the BGM clustering algorithm for this window length.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated herein by the following claims.

## Claims

1. A processor implemented method (200), comprising:
receiving in real time (202), via one or more hardware processors, a first signal and a second signal generated as a neural response of a user to at least one of a plurality of stimuli actions from each of a plurality of channels within a wearable Ear-electroencephalography (Ear-EEG) device worn by the user, wherein the plurality of stimuli actions are indicative of a context from day-to-day activities of the user obtained automatically from the wearable Ear-EEG device, and wherein the second signal is indicative of a resting or a non-resting state of the user;
preprocessing (204), via the one or more hardware processors, one of: (i) the first signal and (ii) the second signal using a plurality of preprocessing techniques to obtain a preprocessed signal;
segmenting (206), via the one or more hardware processors, the preprocessed signal into a plurality of data segments following an onset of the second signal, wherein the plurality of data segments indicate epochs of a plurality of window lengths;
extracting (208), via the one or more hardware processors, a plurality of manifold features from each of the plurality of data segments, wherein the plurality of manifold features support in (i) determining a structure of data and (ii) identifying one or more complex dynamic characteristics of the first signal the second signal;
classifying (210), via the one or more hardware processors, each of the plurality of data segments into (i) a first category and a (ii) a second category based on the plurality of manifold features using an unsupervised clustering model, wherein the first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data; and
selecting (212), via the one or more hardware processors, the plurality of data segments classified into the first category to identify the first set of channels which capture the electroencephalogram (EEG) data for monitoring a status representing health condition of the user.

2. The processor implemented method as claimed in claim 1, wherein the first signal is a continuous signal obtained from the wearable Ear-EEG device.

3. The processor implemented method as claimed in claim 1, wherein the second signal is a modified form of the first signal obtained from the wearable Ear-EEG device when the context from the day-to-day activities of the user is obtained automatically from the wearable Ear-EEG device.

4. The processor implemented method as claimed in claim 1, wherein the second signal is an event based intermittent signal.

5. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive in real time, a first signal and a second signal generated as a neural response of a user to at least one of a plurality of stimuli actions from each of a plurality of channels within a wearable Ear-electroencephalography (Ear-EEG) device worn by the user, wherein the plurality of stimuli actions are indicative of a context from day-to-day activities of the user obtained automatically from the wearable Ear-EEG device, and wherein the second signal is indicative of a resting or a non-resting state of the user;
preprocessing one of: (i) the first signal and (ii) the second signal using a plurality of preprocessing techniques to obtain a preprocessed signal;
segment the preprocessed signal into a plurality of data segments following an onset of the second signal, wherein the plurality of data segments indicate epochs of a plurality of window lengths;
extract a plurality of manifold features from each of the plurality of data segments, wherein the plurality of manifold features support in (i) determining a structure of data and (ii) identifying one or more complex dynamic characteristics of the first signal the second signal;
classify each of the plurality of data segments into (i) a first category and a (ii) a second category based on the plurality of manifold features using an unsupervised clustering model, wherein the first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data; and
select the plurality of data segments classified into the first category to identify the first set of channels which capture the electroencephalogram (EEG) data for monitoring a status representing health condition of the user.

6. The system as claimed in claim 5, wherein the first signal is a continuous signal obtained from the wearable Ear-EEG device.

7. The system as claimed in claim 5, wherein the second signal is a modified form of the first signal obtained from the wearable Ear-EEG device when the context from the day-to-day activities of the user is obtained automatically from the wearable Ear-EEG device.

8. The system as claimed in claim 5, wherein the second signal is an event based intermittent signal.

9. One or more non-transitory machine-readable information storage mediums comprising one or more instructions which when executed by one or more hardware processors cause:
receiving in real time, a first signal and a second signal generated as a neural response of a user to at least one of a plurality of stimuli actions from each of a plurality of channels within a wearable Ear-electroencephalography (Ear-EEG) device worn by the user, wherein the plurality of stimuli actions are indicative of a context from day-to-day activities of the user obtained automatically from the wearable Ear-EEG device, and wherein the second signal is indicative of a resting or a non-resting state of the user;
preprocessing one of: (i) the first signal and (ii) the second signal using a plurality of preprocessing techniques to obtain a preprocessed signal;
segmenting the preprocessed signal into a plurality of data segments following an onset of the second signal, wherein the plurality of data segments indicate epochs of a plurality of window lengths;
extracting a plurality of manifold features from each of the plurality of data segments, wherein the plurality of manifold features support in (i) determining a structure of data and (ii) identifying one or more complex dynamic characteristics of the first signal the second signal;
classifying each of the plurality of data segments into (i) a first category and a (ii) a second category based on the plurality of manifold features using an unsupervised clustering model, wherein the first category indicates a first set of channels from the plurality of channels within the wearable Ear-EEG device which capture an electroencephalogram (EEG) data and the second category indicates a second set of channels from the plurality of channels within the wearable Ear-EEG device which capture a non-electroencephalogram (EEG) data; and
selecting the plurality of data segments classified into the first category to identify the first set of channels which capture the electroencephalogram (EEG) data for monitoring a status representing health condition of the user.

10. The one or more non-transitory machine-readable information storage mediums as claimed in claim 9, wherein the first signal is a continuous signal obtained from the wearable Ear-EEG device.

11. The one or more non-transitory machine-readable information storage mediums as claimed in claim 9, wherein the second signal is a modified form of the first signal obtained from the wearable Ear-EEG device when the context from the day-to-day activities of the user is obtained automatically from the wearable Ear-EEG device.

12. The one or more non-transitory machine-readable information storage mediums as claimed in claim 9, wherein the second signal is an event based intermittent signal.
